(19) **Europäisches Patentamt European Patent Office Office européen des brevets**

(11) **EP 4 094 782 B1**

(12) **EUROPEAN PATENT SPECIFICATION**

(45) Date of publication and mention
of the grant of the patent:
**06.05.2026 Bulletin 2026/19**

(21) Application number: **21744905.7**

(22) Date of filing: **13.01.2021**

(51) International Patent Classification (IPC):
*A61L 9/01* *(2006.01)*    *A61K 8/33* *(2006.01)*
*A61Q 13/00* *(2006.01)*    *A61Q 15/00* *(2006.01)*
*A61L 9/012* *(2006.01)*

(52) Cooperative Patent Classification (CPC):
**A61Q 15/00; A61K 8/33; A61L 9/012; A61Q 13/00;**
A61Q 1/00; A61Q 11/00; A61Q 19/00

(86) International application number:
**PCT/JP2021/000917**

(87) International publication number:
**WO 2021/149566 (29.07.2021 Gazette 2021/30)**

(54) **DEODORANT COMPOSITION**

**DESODORIERENDE ZUSAMMENSETZUNG**

**COMPOSITION DÉODORANTE**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.01.2020 JP 2020006785**

(43) Date of publication of application:
**30.11.2022 Bulletin 2022/48**

(73) Proprietor: **Takasago International Corporation
Tokyo 144-8721 (JP)**

(72) Inventors:
• **NAGASAWA Kazuhiro**
**Hiratsuka-shi, Kanagawa 254-0073 (JP)**
• **KUWAHARA Yukari**
**Hiratsuka-shi, Kanagawa 254-0073 (JP)**

(74) Representative: **Hoffmann Eitle
Patent- und Rechtsanwälte PartmbB
Arabellastraße 30
81925 München (DE)**

(56) References cited:
JP-A- 2001 348 308        JP-A- 2002 504 118
JP-A- S4 953 178          US-A1- 2010 111 889
US-A1- 2017 360 822

**Description**

TECHNICAL FIELD

The present invention relates to a deodorant composition.

BACKGROUND ART

In the related art, chemical deodorants (Patent Literatures 1 and 2) using a natural extract from a plant and sensory deodorants (Patent Literatures 3 and 4) using a blended fragrance have been reported as deodorants used for removing a nitrogen-based malodor, a sulfur-based malodor, and an acid-based malodor.
US 2017/360822 AI describes a malodor neutralizing composition comprising hyaluronic acid or a salt thereof (HA), N-acetyl cysteine or a pharmaceutically or cosmetically acceptable derivative thereof (NAC), and a pharmaceutically or cosmetically acceptable malodor neutralizing agent.
US 2010/111889 AI concerns a malodor control system suitable for use in disposable articles, which may comprise an aldehyde, an ester, an ionone, and optionally a macrocyclic musk. The system may optionally include a perfume.
JP 2001 348308 A relates to a bacteria metabolism inhibitor which contains one or more kinds of compounds selected from 87 kinds of specified perfumes and chemical substances.

CITATION LIST

PATENT LITERATURE

Patent Literature 1: JP-A-S61-206448
Patent Literature 2: JP-A-H11-178907
Patent Literature 3: JP-A-2003-137758
Patent Literature 4: JP-A-2004-018431

SUMMARY OF INVENTION

TECHNICAL PROBLEM

However, for the deodorant using a natural extract, what kind of substance acts as an active ingredient has not been elucidated in many cases. In addition, even if the active ingredient is elucidated, a process for separating these active ingredients is often complicated, and the price of the deodorant becomes expensive, which is not industrially advantageous.
For the deodorant using a blended fragrance, a masking effect is often obtained by strong fragrance, and a user may feel uncomfortable. In addition, although a temporary masking effect can be obtained, the malodor is not fundamentally removed, and therefore, there is a disadvantage that the malodor is felt again over time.
Accordingly, an object of the present invention is to provide a deodorant composition that can chemically deodorize and remove a nitrogen-based malodor of ammonia, amines, or the like, a sulfur-based malodor of mercaptans, sulfides, or the like, and an acid-based malodor of lower fatty acids or the like, and can allow a user to get a fresh feeling sensuously and can impart a fresh feeling.

SOLUTION TO PROBLEM

As a result of studies to solve the above problems, the present inventors have found that a deodorant composition containing both compounds, dihydrocitronellal and hydroxycitronellal has a high deodorization effect both chemically and sensuously, and have completed the present invention.
That is, the present invention includes the following contents [1] to [5].

[1] A deodorant composition containing dihydrocitronellal and hydroxycitronellal.
[2] The deodorant composition according to [1], in which a mass ratio of dihydrocitronellal to hydroxycitronellal is 20:80 to 80:20.
[3] The deodorant composition according to [2], in which the mass ratio is 50:50 to 80:20.
[4] The deodorant composition according to any one of [1] to [3], in which a deodorant target is at least one compound selected from the group consisting of ammonia, amines, mercaptans, sulfides, and acid-based malodorous ingredients selected from the group consisting of acetic acid, propionic acid, normal-butyric acid, isobutyric acid, normal-valeric acid, isovaleric acid, hexanoic acid, heptanoic acid, octanoic acid, 2-methylbutyric acid, 4-methylpentanoic acid, 4-methyl-3-pentenoic acid, 4-methylhexanoic acid, 4-methyl-3-hexenoic acid, 3-methyl-2-hexenoic acid, and 2-ethylhexanoic acid.

[5] A consumer product containing 0.001 mass% to 30 mass% of the deodorant composition according to any one of [1] to [4].

ADVANTAGEOUS EFFECTS OF INVENTION

The deodorant composition of the present invention can chemically deodorize and remove a nitrogen-based malodor of ammonia, amines, or the like, a sulfur-based malodor of mercaptans, sulfides, or the like, and an acid-based malodor of lower fatty acids or the like, and can allow a user to get a fresh feeling sensuously and can impart a fresh feeling.

DESCRIPTION OF EMBODIMENTS

Hereinafter, a deodorant composition of the present invention will be described in detail.

The deodorant composition of the present invention contains both compounds, dihydrocitronellal and hydroxycitronellal as an active ingredient.

As dihydrocitronellal and hydroxycitronellal, commercially available ones may be used, or those obtained by a known production method may be used.

A mass ratio of dihydrocitronellal (A) to hydroxycitronellal (B) in the deodorant composition of the present invention is not strictly limited, and the mass ratio of (A):(B) is preferably 20:80 to 80:20, and more preferably 50:50 to 80:20.

Examples of malodorous ingredients targeted by the deodorant composition of the present invention include nitrogen-based malodorous ingredients such as ammonia, methylamine, ethylamine, trimethylamine, triethylamine, pyridine, ethylpyridine, 3-ethenyl pyridine, pyrazine, skatole, indole, and nicotine; sulfur-based malodorous ingredients such as hydrogen sulfide, methyl mercaptan, ethyl mercaptan, propyl mercaptan, allyl mercaptan, phenyl mercaptan, benzyl mercaptan, dimethyl sulfide, dimethyl disulfide, dimethyl trisulfide, and diallyl sulfide; and acid-based malodorous ingredients such as acetic acid, propionic acid, normal-butyric acid, isobutyric acid, normal-valeric acid, isovaleric acid, hexanoic acid, heptanoic acid, octanoic acid, 2-methylbutyric acid, 4-methylpentanoic acid, 4-methyl-3-pentenoic acid, 4-methylhexanoic acid, 4-methyl-3-hexenoic acid, 3-methyl-2-hexenoic acid, and 2-ethylhexanoic acid.

The deodorant composition of the present invention also has a deodorization effect on a composite malodor generated by the above-described malodorous ingredients and other malodorous ingredients.

Examples of the composite malodorous ingredient targeted by the deodorant composition of the present invention include human body-related odors such as a body odor, a smell of sweat, an underarm odor, a foot odor, a scalp odor, a middle fat odor, and an age-related body odor, clothing-related odors such as an odor of damp-dried clothes, an odor of left laundry, and an odor adhering to clothes, a tobacco odor, a toilet odor, an entrance odor, an indoor odor, a bedroom odor, a shoe rack odor, a swill odor, a bathroom odor, an odor adhering to fibers of curtain, bedding, or the like, a refrigerator odor, an air conditioner odor, a diaper odor, an odor caused by a disease, a long-term care related odor, a pet related odor, and the like.

If necessary, the deodorant composition of the present invention can be used in combination with other ingredients such as deodorants, oils and fats, waxes, hydrocarbons, alcohols, glycols, glycol ethers, glycol esters, ethers, esters, ketones, fatty acids, surfactants, flavor or fragrances, and the like. The other ingredients may be used alone or in combination of two or more kinds thereof.

Examples of the deodorants include $\alpha$-cyclodextrin, $\beta$-cyclodextrin, $\gamma$-cyclodextrin, hydroxypropyl-$\beta$-cyclodextrin, methyl-$\beta$-cyclodextrin, maltosyl-$\beta$-cyclodextrin, tannins, chlorogenic acid, gallic acid, catechins, flavones, isoflavones, flavanols, flavonols, lauryl methacrylates, geranylcrotonates, zinc ricinoleate, zinc undecylenate, silver undecylenate, aluminum sulfate, aluminum acetate, chlorohydroxyaluminum, sodium hydrogen carbonate, magnesium sulfate, calcium carbonate, titanium oxide, alum, activated carbon, charcoal, bamboo charcoal, zeolite, bentonite, montmorillonite, and the like.

Examples of the oils and fats include avocado oil, almond oil, olive oil, hardened oil, coconut oil, castor oil, hardened castor oil, soybean oil, sesame oil, and the like.

Examples of the waxes include candelilla wax, jojoba oil, beeswax, and the like.

Examples of the hydrocarbons include normal paraffins, isoparaffins, paraffins, squalanes, liquid paraffins, vaseline, toluene, xylene, mineral spirit, isohexane, normal decane, normal heptane, and the like.

Examples of the alcohols include ethyl alcohol, isopropyl alcohol, butyl alcohol, isostearyl alcohol, 1,3-butanediol, and the like.

Examples of the glycols include propylene glycol, dipropylene glycol, hexylene glycol, butylene glycol, glycerin, and the like.

Examples of the glycol ethers include propylene glycol monomethyl ether, dipropylene glycol monomethyl ether, dipropylene glycol dimethyl ether, 3-methoxy-3-methyl-1-butanol, and the like.

Examples of the glycol esters include propylene glycol methyl ether acetate, propylene glycol diacetate, dipropylene glycol methyl ether acetate, and the like.

Examples of the ethers include butyl carbitol, 2-methoxyethanol, and the like.

Examples of the esters include benzyl benzoate, triethyl citrate, decyl oleate, diethyl phthalate, and the like.

Examples of the ketones include acetone, diacetone alcohol, cyclohexanone, and the like.

Examples of the fatty acids include isostearic acid, undecylenic acid, oleic acid, stearic acid, palmitic acid, myristic acid, coconut oil fatty acids, and the like.

Examples of the surfactant include a nonionic surfactant, an anionic surfactant, a nonionic surfactant, an amphoteric surfactant, and the like.

Examples of the flavor or fragrances include natural essential oils such as lemon oil, orange oil, lime oil, bergamot oil, lavandula hybrida oil, lavender oil, geranium oil, rose oil, and sandalwood oil; hydrocarbons such as $\alpha$-pinene, $\beta$-pinene, limonene, and p-cymene; aliphatic alcohols such as octanol and p-tert-butylcyclohexanol; terpene-based alcohols such as menthol, citronellol, and geraniol; aromatic alcohols such as benzyl alcohol and phenylethyl alcohol; aliphatic aldehydes; terpene-based aldehydes; aromatic aldehydes; acetals; open-chain ketones; cyclic ketones such as damascones, $\beta$-ionone, and methyl ionone; terpene-based ketones such as carvone, menthone, isomenthone, and camphor; aromatic ketones such as acetophenone and raspberry ketone; ethers such as dibenzyl ether; oxides such as linalool oxide and rose oxide; musks such as cycropentadecanolide and cyclohexadecanolide; lactones such as $\gamma$-nonalactone, $\gamma$-undecalactone, and coumarin; aliphatic esters such as acetates and propionates; aromatic esters such as benzoates and phenylacetates; and the like.

The deodorant composition of the present invention can be combined with a known carrier. Examples of the carrier include gas carriers such as LPG, liquid carriers such as water, alcohols, glycols, and flavor or fragrance compositions, and solid carriers such as calcium silicate, silica gel, gelling agents, and thickeners.

Furthermore, the deodorant composition of the present invention can be used in combination with additives such as a cooling agent, a warming agent, a stimulant, a repellent agent, an insect repellent, an antibacterial agent, an antifungal agent, an antirust agent, a preservative, an antioxidant, a light stabilizer, a pH adjuster, a pigment, an ultraviolet absorber, an antifoaming agent, an antistatic agent, a gelling agent, a thickener, and the like.

The deodorant composition of the present invention can be used as it is as a final product such as a deodorant and an odor remover.

Alternatively, the deodorant composition of the present invention is preferably used by being added to consumer products such as aromatic products, house hold products, toiletry products, and cosmetics.

Examples of the aromatic products include liquid aromatics, powdery aromatics, gel aromatics, aerosol aromatics, mist aromatics, impregnated aromatics, paper aromatics, lead diffusers, incense stick, plastic molded aromatics, permeable film aromatics, water-absorbent polymer aromatics, and the like.

Examples of the house hold products include sprays for clothes, mists for clothes, detergents for clothes, softeners for clothes, bleaching agents for clothes, wrinkle removing agents, detergents for tableware, cleaning agents for a drain outlet, cleaning agents for a floor, cleaning agents for a washing machine, cleaning agents for a bathroom, cleaning agents for a toilet, and the like.

Examples of the toiletry products include body washers, deodorants, bath agents, oral care products, sanitary products, paper diapers, shampoos, rinses, conditioners, treatments, hair tonics, hair styling agents, hair growing agents, bleaching agents, permanent agents, and hair coloring agents.

Examples of the cosmetics include skin lotions, milky lotions, creams, soaps, liquid soaps, facial washes, sunscreens, antiperspirants, lipsticks, foundations, and the like.

By using the deodorant composition of the present invention, these consumer products can be given a deodorization effect and an odor removal effect, preferably, an effect of removing a nitrogen-based malodor of ammonia, amines, or the like, a sulfur-based malodor of mercaptans, sulfides, or the like, and an acid-based malodor of lower fatty acids or the like.

A content of the deodorant composition of the present invention in the consumer product may be appropriately determined depending on the form, application, and the like of the consumer product, and is not particularly limited. However, the content of the deodorant composition of the present invention is preferably in the range of 0.001 mass% to 30 mass%, and more preferably in the range of 0.01 mass% to 10 mass%, with respect to the total mass of the consumer product.

The deodorant composition of the present invention can be used for deodorization or odor removal, for example, garbage, clothes, body odor, refrigerators, chest of drawers, closets, cabinets, indoor, vehicle interiors, toilets, bathrooms, pet products, factory interiors, industrial waste liquids, air purifiers, air conditioners, deodorizing machines, filters for air blowers or air dischargers, sewage treatment plants, livestock barns, and dust treatment plants.

The deodorant composition of the present invention is excellent in the deodorization effect of a nitrogen-based malodor of ammonia, amines, or the like, a sulfur-based malodor of mercaptans, sulfides, or the like, and an acid-based malodor of lower fatty acids or the like. Therefore, by applying the deodorant composition of the present invention to a smell of sweat, a fatigue-related odor, a toilet odor, a tobacco odor, a waste odor, an indoor odor, a pet odor, a smell of breath, a swill odor, a drain outlet odor, a refrigerator odor, an odor caused by stress, an underarm odor, a foot odor, a scalp odor, an odor of damp-dried clothes, and the like, which contain a large number of malodorous ingredients and are generated from a daily living environment, the deodorization effect functions and the generation of the malodor can be effectively prevented.

The deodorant composition of the present invention can be obtained, for example, by mixing and stirring ingredients, and heating or the like may be performed as desired. The content of the ingredients may be appropriately adjusted.

[Examples]

Hereinafter, the present invention will be described in more detail with reference to Examples, but the present invention is not limited thereto.

(Preparation 1 of Deodorant Composition)

Deodorant compositions of Examples and Comparative Examples were prepared according to a formulation shown in Table 1. The blending amount in the table is expressed in mass%.

[Table 1]

| Blending ingredients | Comparative Examples | | | | Examples | |
|---|---|---|---|---|---|---|
| | 1 | 2 | 3 | 4 | 1 | 2 |
| Persimmon extract deodorant | 100 | - | - | - | - | - |
| Amyris oil | - | 2 | - | - | - | - |
| Triethylcitrate | - | 98 | 80 | 80 | 80 | 60 |
| Hydroxycitronellal | - | - | 20 | - | 10 | 20 |
| Dihydrocitronellal | - | - | - | 20 | 10 | 20 |
| Total | 100 | 100 | 100 | 100 | 100 | 100 |

Test Example 1: <Chemical Deodorization Effect Test on Trimethylamine>

In a petri dish type container, 0.1 g of a sample of each of Comparative Examples 1 to 4 and Examples 1 and 2 was placed, and the petri dish type container was placed in a 2 L sampling bag (Smart Bag, PA, Model AAK-2, manufactured by GL Sciences Inc.), followed by sealing the sampling bag. In addition, a 2 L sampling bag that was sealed after only a petri dish type container was placed therein was regarded as a control. Subsequently, air in each of the sampling bags was removed, and 2 L of a trimethylamine gas of 40 ppm prepared in advance was injected into each of the sampling bags, followed by sealing the sampling bags again. After being allowed to stand for 2 hours, a concentration of trimethylamine was measured with a detection tube (for amines, No. 180, manufactured by Gastec Corporation), and a deodorization rate was determined by the following formula. The results are shown in Table 2.

(Calculation Formula of Deodorization Rate)

$$\text{Deodorization rate (\%)} = \{(C - S)/C\} \times 100$$

S: a concentration of an odiferous gas in a sampling bag containing a test sample
C: a concentration of an odiferous gas in a sampling bag as a control

[Table 2]

|  | Comparative Examples | | | | Examples | |
|---|---|---|---|---|---|---|
|  | 1 | 2 | 3 | 4 | 1 | 2 |
| Deodorization rate after 2 hours (%) | 3 | 3 | 44 | 7 | 100 | 100 |

As shown in Table 2, the deodorization effect was insufficient in Comparative Examples 1 and 2 as the prior art and Comparative Examples 3 and 4 containing only either dihydrocitronellal or hydroxycitronellal. In contrast, in Examples 1 and 2, 100% of trimethylamine could be deodorized and removed.

Test Example 2: <Chemical Deodorization Effect Test on Methyl Mercaptan>

In a petri dish type container, 0.2 g of a sample of each of Comparative Examples 1 to 4 and Examples 1 and 2 was placed, and the petri dish type container was placed in a 2 L sampling bag (Smart Bag, PA, Model AAK-2, manufactured by GL Sciences Inc.), followed by sealing the sampling bag. In addition, a 2 L sampling bag that was sealed after only a petri dish type container was placed therein was regarded as a control. Subsequently, air in each of the sampling bags was removed, and 2 L of a methyl mercaptan gas of 1 ppm prepared in advance was injected into each of the sampling bags, followed by sealing the sampling bags again. After being allowed to stand for 3 hours, a concentration of methyl mercaptan was measured with a detection tube (for mercaptans, No. 70L, manufactured by Gastec Corporation), and a deodorization rate was determined in the same manner as in Test Example 1. The results are shown in Table 3.

[Table 3]

|  | Comparative Examples | | | | Examples | |
|---|---|---|---|---|---|---|
|  | 1 | 2 | 3 | 4 | 1 | 2 |
| Deodorization rate after 3 hours (%) | 0 | 0 | 5 | 40 | 100 | 100 |

As shown in Table 3, the deodorization effect was insufficient in Comparative Examples 1 and 2 as the prior art and Comparative Examples 3 and 4 containing only either dihydrocitronellal or hydroxycitronellal. In contrast, in Examples 1 and 2, 100% of methyl mercaptan could be deodorized and removed.

Test Example 3: <Chemical Deodorization Effect Test on Acetic Acid>

In a petri dish type container, 0.2 g of a sample of each of Comparative Examples 1 to 4 and Examples 1 and 2 was placed, and the petri dish type container was placed in a 2 L sampling bag (Smart Bag, PA, Model AAK-2, manufactured by GL Sciences Inc.), followed by sealing the sampling bag. In addition, a 2 L sampling bag that was sealed after only a petri dish type container was placed therein was regarded as a control. Subsequently, air in each of the sampling bags was removed, and 2 L of an acetic acid gas of 50 ppm prepared in advance was injected into each of the sampling bags, followed by sealing the sampling bags again. After being allowed to stand for 3 hours, a concentration of acetic acid was measured with a detection tube (for acetic acid, No. 81, manufactured by Gastec Corporation), and a deodorization rate was determined in the same manner as in Test Example 1. The results are shown in Table 4.

[Table 4]

|  | Comparative Examples | | | | Examples | |
|---|---|---|---|---|---|---|
|  | 1 | 2 | 3 | 4 | 1 | 2 |
| Deodorization rate after 3 hours (%) (%) | 12 | 10 | 34 | 30 | 100 | 100 |

As shown in Table 4, the deodorization effect was insufficient in Comparative Examples 1 and 2 as the prior art and Comparative Examples 3 and 4 containing only either dihydrocitronellal or hydroxycitronellal. In contrast, in Examples 1 and 2, 100% of acetic acid could be deodorized and removed.

(Preparation 2 of Deodorant Composition)

Deodorant compositions of Examples and Comparative Examples were prepared according to a formulation shown in Table 5. The blending amount in the table is expressed in mass%.

[Table 5]

| Blending ingredients | Comparative Examples | | | | Examples | | |
|---|---|---|---|---|---|---|---|
| | 1 | 2 | 5 | 6 | 3 | 4 | 5 |
| Persimmon extract deodorant | 100 | - | - | - | - | - | |
| Amyris oil | - | 2 | - | - | - | - | |
| Triethylcitrate | - | 98 | - | - | - | - | - |
| Hydroxycitronellal | - | - | 100 | - | 80 | 50 | 20 |
| Dihydrocitronellal | - | - | - | 100 | 20 | 50 | 80 |
| Total | 100 | 100 | 100 | 100 | 100 | 100 | 100 |

Model toilet odors were prepared according to a formulation shown in Table 6. The blending amount in the table is expressed in mass%.

[Table 6]

| Types of malodor | Blending ingredients | Blending amount |
|---|---|---|
| Nitrogen-based malodor | 28% ammonia water | 0.05 |
| | Indole | 0.05 |
| Sulfur-based malodor | Dimethyl sulfide | 0.01 |
| | Dimethyl disulfide | 0.02 |
| Acid-based malodor | Propionic acid | 0.02 |
| | Butyric acid | 0.03 |
| | Isovaleric acid | 0.02 |
| Dilution solvent | Dipropylene glycol | 99.8 |
| Total | | 100 |

Test Example 4: <Sensory Deodorization Effect Test on Model Toilet Odor>

Into a cotton ball having a diameter of 10 mm, 10 μL of a sample of each of Comparative Examples 1, 2, 5, and 6 and Examples 3 to 5 was absorbed, and the cotton ball was placed in a 20 mL narrow mouth container. Subsequently, 10 μL of a model toilet odor was absorbed into a newly prepared cotton ball, and the cotton ball was placed in the narrow mouth container in which the cotton ball in each of Comparative Examples and Examples was placed, and the narrow mouth container was capped. In addition, a narrow mouth container that is capped after only a cotton ball absorbing 10 μL of a model toilet odor was placed therein was used as a control. After being allowed to stand for 2 hours, the cap was opened, and sensory evaluation was performed from the container opening portion according to the following evaluation criteria. A relative evaluation was performed by setting an evaluation score of the control to -2.0, and an average value of the evaluation scores given by six professional evaluation panelists was determined. The results of the sensory evaluation are shown in Table 7.

(Evaluation Criteria)

-3: Very uncomfortable
-2: Uncomfortable
-1: Slightly uncomfortable
0: Neither comfortable nor uncomfortable
1: Slightly comfortable

2: Comfortable
3: Very comfortable

[Table 7]

| | Control | Comparative Examples | | | | Examples | | |
|---|---|---|---|---|---|---|---|---|
| | | 1 | 2 | 5 | 6 | 3 | 4 | 5 |
| Evaluation score after 2 hours | -2.0 | -2.3 | -2.3 | -1.3 | -0.3 | 0.3 | 1.3 | 1.3 |

As shown in Table 7, Examples 3 to 5 exhibited a high sensory deodorization effect on the model toilet odor as compared with Comparative Examples 1 and 2 as the prior art, and Comparative Examples 5 and 6 containing only either dihydrocitronellal or hydroxycitronellal.

When Example 3 was compared with Examples 4 and 5, the deodorant composition having a mass ratio of dihydrocitronellal to hydroxycitronellal of 50:50 to 80:20 exhibited a higher sensory deodorization effect than the deodorant composition having a mass ratio of dihydrocitronellal to hydroxycitronellal out of the range.

## Claims

1. A deodorant composition comprising dihydrocitronellal and hydroxycitronellal.

2. The deodorant composition according to claim 1, wherein a mass ratio of dihydrocitronellal to hydroxycitronellal is 20:80 to 80:20.

3. The deodorant composition according to claim 2, wherein the mass ratio is 50:50 to 80:20.

4. The deodorant composition according to any one of claims 1 to 3, wherein a deodorant target is at least one compound selected from the group consisting of ammonia, amines, mercaptans, sulfides, and acid-based malodorous ingredients selected from the group consisting of acetic acid, propionic acid, normal-butyric acid, isobutyric acid, normal-valeric acid, isovaleric acid, hexanoic acid, heptanoic acid, octanoic acid, 2-methylbutyric acid, 4-methylpentanoic acid, 4-methyl-3-pentenoic acid, 4-methylhexanoic acid, 4-methyl-3-hexenoic acid, 3-methyl-2-hexenoic acid, and 2-ethylhexanoic acid.

5. A consumer product comprising 0.001 mass% to 30 mass% of the deodorant composition according to any one of claims 1 to 4.

## Patentansprüche

1. Deodorantzusammensetzung, umfassend Dihydrocitronellal und Hydroxycitronellal.

2. Deodorantzusammensetzung gemäß Anspruch 1, wobei das Massenverhältnis von Dihydrocitronellal zu Hydroxycitronellal 20:80 bis 80:20 beträgt.

3. Deodorantzusammensetzung gemäß Anspruch 2, wobei das Massenverhältnis 50:50 bis 80:20 beträgt.

4. Deodorantzusammensetzung gemäß mindestens einem der Ansprüche 1 bis 3, wobei ein Deodorantziel mindestens eine Verbindung ist, die ausgewählt ist aus der Gruppe bestehend aus Ammoniak, Aminen, Mercaptanen, Sulfiden und säurebasierten übelriechenden Inhaltsstoffen, die ausgewählt sind aus der Gruppe bestehend aus Essigsäure, Propionsäure, normaler Buttersäure, Isobuttersäure, normaler Valeriansäure, Isovaleriansäure, Hexansäure, Heptansäure, Octansäure, 2-Methylbuttersäure, 4-Methylpentansäure, 4-Methyl-3-pentensäure, 4-Methylhexansäure, 4-Methyl-3-hexensäure, 3-Methyl-2-hexensäure und 2-Ethylhexansäure.

5. Verbraucherprodukt, umfassend 0,001 Massen-% bis 30 Massen-% der Deodorantzusammensetzung gemäß mindestens einem der Ansprüche 1 bis 4.

**Revendications**

1. Composition déodorante comprenant dihydrocitronellal et hydroxycitronellal.

2. Composition déodorante selon la revendication 1, dans laquelle un rapport massique de dihydrocitronellal à hydroxycitronellal est de 20:80 à 80:20.

3. Composition déodorante selon la revendication 2, dans laquelle le rapport massique est de 50:50 à 80:20.

4. Composition déodorante selon l'une quelconque des revendications 1 à 3, dans laquelle la cible de désodorisation est au moins un composé choisi dans le groupe constitué de l'ammoniac, d'amines, de mercaptans, de sulfures, et de composés malodorants à base d'acide choisis dans le groupe constitué de l'acide acétique, de l'acide propionique, de l'acide butyrique, de l'acide isobutyrique, de l'acide valérique, de l'acide isovalérique, de l'acide hexanoïque, de l'acide heptanoïque, de l'acide octanoïque, de l'acide 2-méthylbutanoïque, de l'acide 4-méthylpentanoïque, de l'acide 4-méthyl-3-penténoïque, de l'acide 4-méthylhexanoïque, de l'acide 4-méthyl-3-hexénoïque, de l'acide 3-méthyl-2-hexénoïque, et de l'acide 2-éthylhexanoïque.

5. Produit de consommation comprenant de 0,001 % en masse à 30 % en masse de la composition déodorante selon l'une quelconque des revendications 1 à 4.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2017360822 A1
- US 2010111889 A1
- JP 2001348308 A
- JP S61206448 A
- JP H11178907 A
- JP 2003137758 A
- JP 2004018431 A